# EUROPEAN PATENT SPECIFICATION

(11) **EP 2 858 583 B1**
(45) Date of publication and mention of the grant of the patent: **03.08.2016**
(21) Application number: 13734850.4
(22) Date of filing: 22.05.2013
(51) Int. Cl.: A61B 17/66

(54) **FLEXIBLE DRIVE ASSEMBLY AND BONE DISTRACTION APPARATUS IN WHICH THE DRIVE ASSEMBLY IS USED**
FLEXIBLE ANTRIEBSANORDNUNG UND KNOCHENDISTRAKTIONSVORRICHTUNG MIT DER DARIN VERWENDETEN ANTRIEBSANORDNUNG
ENSEMBLE D'ENTRAÎNEMENT FLEXIBLE ET APPAREIL DE DISTRACTION OSSEUSE UTILISANT LEDIT ENSEMBLE D'ENTRAÎNEMENT

(30) Priority: 07.06.2012 ZA 201204176
(43) Date of publication of application: 15.04.2015
(73) Proprietor: Southern Implants (Pty) Ltd., 0157 Centurion (ZA)
(72) Inventor: GERHARDT, Thomas, 0157 Centurion (ZA); PIETERSE, Richard Kleynhans, 0157 Centurion (ZA)
(74) Representative: Parry, Simon James
(86) International application number: PCT/IB2013/054225
(87) International publication number: WO 2013/182936

(56) References cited:
- WO-A2-2006/073581
- US-A- 6 113 599
- US-A1- 2010 193 567

## Description

### BACKGROUND TO THE INVENTION

THIS invention relates to a bone distraction apparatus in which a flexible drive assembly is used.

Distraction osteogenesis, also known as callus distraction, callotasis or osteo-distraction, is a surgical process used to reconstruct skeletal deformities and lengthen bones of the body. In this process, a corticotomy is used to fracture the bone which is to be lengthened. In the corticotomy, the cortex of the bone is cut or fractured, leaving surrounding tissues intact. After the corticotomy, the two cortical bone ends are gradually moved apart, i.e. distracted, during a distraction phase, allowing new bone to form in the gap between the separated bone ends. When the required amount of distraction has taken place, a consolidation phase follows in which the bone continues healing.

Distraction technology as outlined above was primarily used in orthopaedics, but is now also used by maxillofacial surgeons to correct deformities of the jaw such as microagnathia (undersize jaw) and midface and fronto-orbital hypoplasia in patients with craniofacial deformities.

A known facial distraction device, available from KLS Martin of the USA, has small bone attachment plates engaged in threaded manner with a power screw forming part of a distraction component. The bone attachment plates are fixed, for example by means of screws, to the bone ends which are to be distracted. During distraction, the power screw is rotated through a predetermined angle at regular intervals in order to drive the bone plates apart, and hence distract the bone ends, through a predetermined distance. In a typical example, the power screw may be rotated one full turn each day in order to distract the bone ends by 1 mm per day.

Given *the* limited space available in the area of the human jaw, typically the mandible, and the natural curvatures present in the jaw anatomy, it is necessary to employ a rotary drive to transmit rotary torque to the power screw from a driving tool such as a wrench. In the KLS Martin device, the flexible drive is provided by a number of tightly coiled springs of progressively smaller diameter located one inside the other. Opposite ends of the spring assembly are connected respectively to the power screw of the distraction component and to a formation engagable by the driving tool.

The spring assembly used in the KLS Martin apparatus has a number of disadvantages including the following:
1. Given the ability of a coil spring to wind up more tightly, the rotation applied by the driving tool may not be fully transmitted to the power screw. It is accordingly difficult to ensure that the power screw has rotated through the correct angle.
2. The spring assembly will yield if torque greater than a predetermined value is applied to it.
3. The spring assembly cannot transmit any significant torque in a reverse direction. Application of reverse torque will merely cause the turns of the springs to open up.
4. It is difficult if not impossible to vary the length of the spring assembly.
5. The resilience of the spring assembly will always tend to straighten it unless an internal, bendable wire is included to maintain a desired angulation of the assembly.
6. The ends of the spring assembly are permanently welded to the power screw and driving tool engagement formation. Strong welds are difficult to achieve.
7. The permanent connection of the spring assembly to the distraction component means that the spring assembly must be left in position, typically embedded in the soft tissue on the buccal side of the anterior mandible, during the normal healing period of anywhere between six and ten weeks. The continued presence of the assembly can compromise hygiene during this period, as bacterial infection can quite easily migrate along the length of the assembly.
8. As the spring assembly can incorporate as many as twelve springs of very small diameter, it is costly and difficult to manufacture.

Document US6113599A discloses a bone distraction mechanism with an articulated flexible drive shaft.

The present invention seeks to provide a bone distraction apparatus which addresses at least some of the problems outlined above.

### SUMMARY OF THE INVENTION

According to the invention there is provided a bone distraction apparatus including a flexible drive assembly comprising a plurality of identical drive elements connected to one another in series, each drive element having a body with a central axis and male and female ends, the male end including a laterally projecting rib of non-circular cross-section and the female end defining a socket with a laterally extending groove therein which is of non-circular cross-section, wherein the drive elements are connected to one another in series by location of the male end of one element in the series in the socket at the female end of a neighbouring element in the series with the rib of the male end received as a snap fit in the groove of the socket, the male ends and sockets being configured to allow limited pivotal movement to take place between adjacent, connected elements, in each case about an axis transverse to the central axes of the elements, while also allowing the elements to transmit rotational torque in either direction from one to the other.

In the preferred embodiment the male end of each drive element has oppositely inclined flanks on either side of the projecting rib, the flanks defining a non-circular cross-section. The flank on one side of the rib may be convex and the flank on the other side of the rib may be concave.

The rib and flanks of the male end of each drive element, and the socket and groove of the female end of each drive element may have a polygonal, preferably hexagonal, cross-section.

Facets of the polygonal cross-section of the socket may be convex.

Typically transverse dimensions of the socket and groove are greater than corresponding transverse dimensions of the rib and flanks by an amount which allows pivotal movement to take place between connected elements while still ensuring rotational interference between the elements for torque transmission purposes.

In the preferred embodiment each element has an axial passage through it, the axial passages of the individual elements combining to form a continuous passage through the connected elements of the drive assembly. With this feature, the drive assembly can include an elongate, bendable member, preferably of wire, which extends through the continuous passage to maintain a desired angulation of .the assembly.

The drive elements may be configured such that each element in the series can be axially misaligned by at least 5°, preferably about 6.5°, relative to adjacent elements.

The apparatus typically includes a distraction component including a rotatable power screw which has respective ends carrying threads of opposite hand and bone attachment members engaged in threaded manner with the respective ends of the power screw such that rotation of the power screw moves the bone attachment members towards or away from one another, and wherein a first, distal end of the flexible drive assembly is connected in a rotation-transmitting manner to the power screw.

Typically the opposite, proximal end of the flexible drive assembly carries a formation engagable by a rotary drive tool, whereby the drive tool can be operated to rotate the drive assembly, and hence the power screw, via the drive assembly.

The apparatus may include a coupling means which connects the flexible drive assembly to the power screw in a manner which allows rotational torque to be transmitted to the power screw in a first direction corresponding to distraction of the bone attachment members, but which disconnects the drive assembly from the power screw if rotational torque of predetermined magnitude is applied to the drive assembly in an opposite, second direction. In the preferred embodiment the coupling means includes a coupler connectable to an end of the power screw, a connection element at the distal end of the drive assembly to which an endmost one of the drive elements is connectable and which is itself connectable to the coupler in a manner to transmit torque in the first direction and which disconnects from the coupler when torque of the predetermined magnitude is applied to the drive assembly in the second direction. It is also preferred that the coupler includes a threaded socket at a distal end thereof into which one end of the power screw is threaded in order to connect the coupler to the power screw at a threaded connection, that the threaded connection is arranged to tighten when torque corresponding to distraction of the bone attachment members is applied, and that the coupler and connection member make threaded engagement with one another, one of them including a radial rib which snaps into a radial groove in the other of them when they are threaded together, and the configuration being such that the rib unsnaps from the recess when the predetermined magnitude of torque is applied in the second direction.

### BRIEF DESCRIPTION OF THE DRAWINGS

The invention will now be described in more detail, by way of example only, with reference to the accompanying drawings in which:
- **Figure 1**: shows a perspective view of a bone distraction apparatus according to the invention, the bone distraction apparatus incorporating a flexible drive assembly;
- **Figure 2**: shows a side view of the apparatus seen in Figure 1;
- **Figure 3**: shows a cross-sectional view of a coupling means;
- **Figure 4**: shows an enlarged cross-sectional view of the encircled area in Figure 2;
- **Figure 5**: shows a perspective view of a single drive element of the flexible drive assembly;
- **Figure 6**: shows another perspective view of the drive element seen in Figure 5;
- **Figure 7**: shows a cross-sectional view of the drive element seen in Figure 5;
- **Figure 8**: shows a side view of the drive element seen in Figure 5;
- **Figure 9**: illustrates the distraction apparatus of the invention in use; and
- **Figure 10**: shows how drive elements can be disconnected from one another.

### SPECIFIC DESCRIPTION

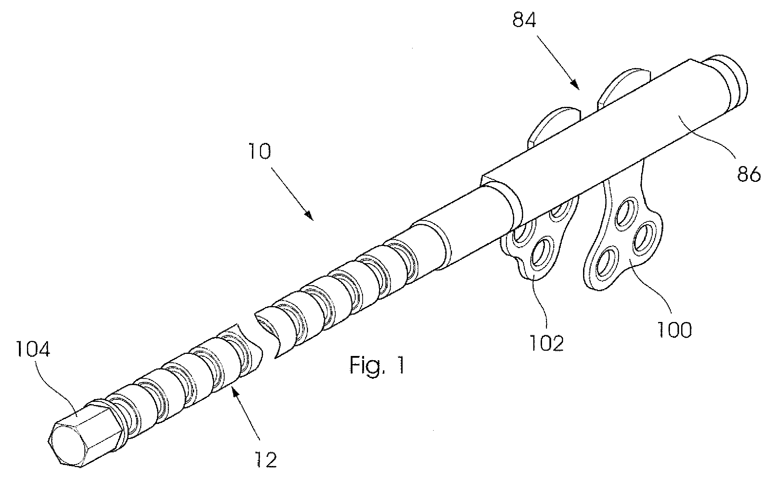

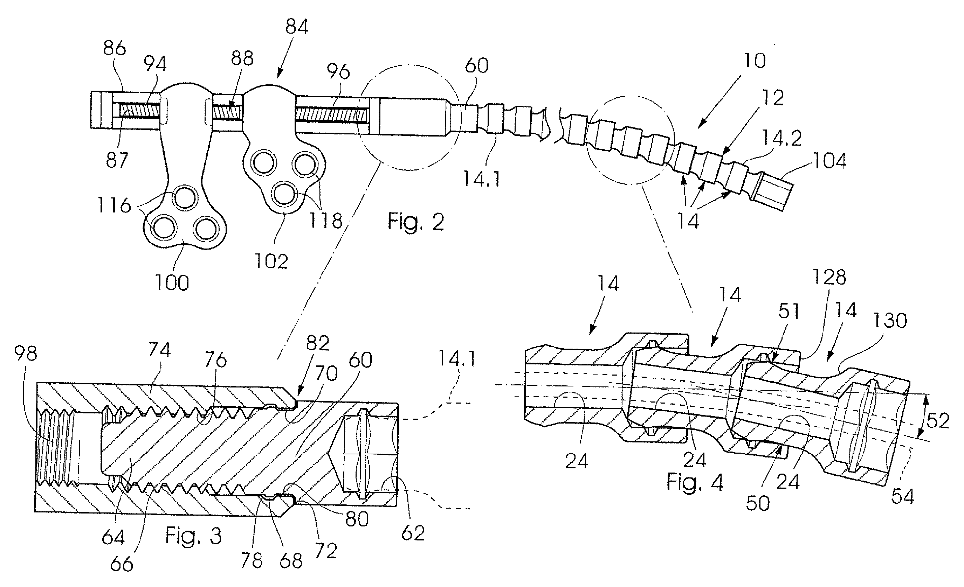

The bone distraction apparatus 10 seen in Figures 1 and 2 includes a flexible drive assembly indicated generally with the numeral 12.

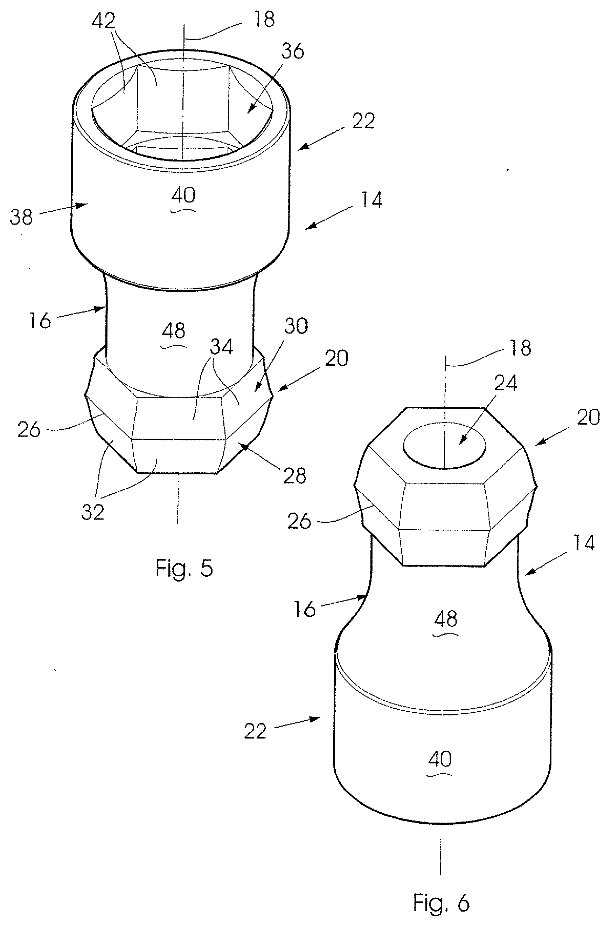

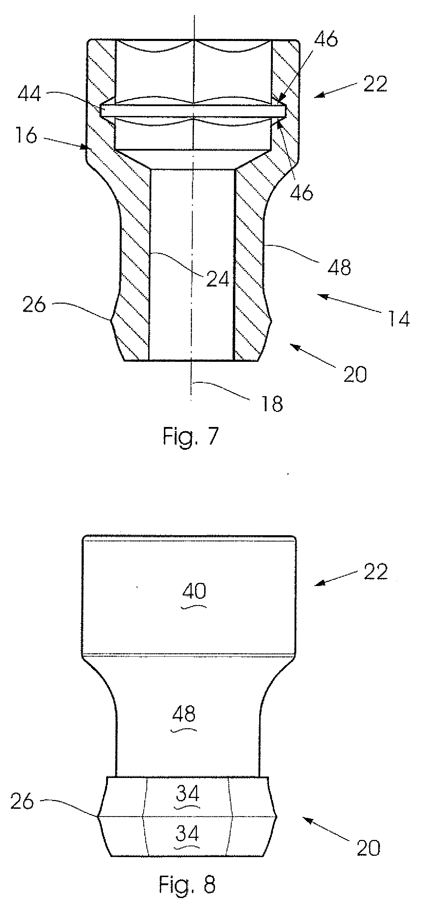

The flexible drive assembly includes a number of identical, discrete drive elements 14, one of which is illustrated in Figures 5 to 8. The element 14 has a one-piece body 16, typically of grade 5 titanium, with a central axis 18, a male end 20 and a female end 22. The body 16 is hollow by virtue of an axial passage 24 extending through it from end to end.

At the enlarged male end, the drive element includes a laterally projecting rib 26 and flanks 28 and 30 which are inclined in opposite directions relative to the rib. The rib and both flanks have a polygonal, in the illustrated case, hexagonal shape in cross-section. The facets 32 of the flank 28 are slightly convex and the facets 34 of the flank 30 are slightly concave.

At the female end, the drive element includes a socket 36 formed in an enlarged end section 38 which has an external surface 40 of round cylindrical shape. The socket has generally a polygonal, in this case hexagonal, shape in cross-section. Each facet 42 of the hexagonal shape has a convexly curved shape. An undercut groove 44 with inclined flanks 46 is formed in the socket as shown.

The male and female ends of the drive element 14 are connected to another by a relatively slender waist 48 of circular cross-section.

The drive elements 14 are connected to one another in series to form the flexible drive assembly 12. Each connection is made by generally aligning the facets of the male end of one element with the facets of the socket 36 of the next element, and pushing the male end into the socket with sufficient force to cause the rib 26 to snap into the groove 44.

The cross-sectional dimensions of the socket are somewhat greater than those of the male end received in the socket. Despite this, the rib 26 is held captive in the groove 44 with sufficient security to prevent the elements from being pulled axially apart from one another without application of substantial force. The greater dimensions of the socket also allow some pivotal movement to take place between the connected elements about an axis transverse to the central axes 18 of the drive elements 14, i.e. such that the axes 18 go out of alignment with one another. This is illustrated in Figure 4 which shows each of the elements pivoted to a maximum angulation relative to the next element. The position of maximum angulation is reached when there is abutment between the waist 48 of one element with the mouth of the socket 36 of the next element, as indicated by the numeral 50. It will be noted that the rib 26 is still located in the groove 44 even at this position of maximum angulation. There is however no contact between the rib 26 and the wall of the groove at the location 51.

Typically, each element will be able to pivot through a maximum angle of at least 5° relative to the next element. In the illustrated embodiment, the maximum angle 52 is approximately 6.5°.

It will be understood that the allowed angulation at each connection between two drive elements can be summed to provide substantial total angulation over the whole length of the drive assembly 12.

As also shown in Figure 4, the passages 24 of the connected elements 14 form a continuous passage. The flexible drive assembly includes a bendable elongate member, in the illustrated embodiment a length of titanium wire 54, which extends within the passage for the full length of the passage. When the drive assembly is flexed to a desired non-linear or curved shape as shown in, for instance, Figure 2, the wire 54 bends accordingly and thereafter serves to maintain the selected curvature. It will be understood that in the absence of the wire, the free pivotal movement which can be take place between adjacent elements 14 means that there is nothing to hold any particular shape of the drive assembly. It will also be understood that the drive assembly can be reshaped as required merely by rebending the wire.

The endmost drive element 14.1, at the distal end of the flexible drive assembly, is connected to a connection element 60. This element has a socket 62 which is geometrically similar to the sockets 36 of the drive elements. The endmost drive element 14 makes a snap fit connection with the connection element 60 in the same way that the drive elements are connected to one another.

The opposite end 64 of the connection element 60 is formed with an external thread 66. At the proximal end of the thread 66 there is a radial, outwardly facing rib 68, a groove 70 and a shoulder 72.

The distraction apparatus also includes a coupler 74 formed at one end with an internal thread 76 complemental to the thread 66. Towards the opposite end of the coupler there is a groove 78 and an inwardly facing rib 80.

During assembly of the distraction apparatus, the threaded end 64 of the connection element is threaded into the coupler. When the thread 66 is fully threaded into the thread 76, the rib 68 snaps into the groove 78 and the shoulder 72 comes into abutment with the end of the coupler as indicated by the numeral 82.

The distraction apparatus 10 also includes a distraction component 84 which includes a housing 86 formed with a longitudinal slot 87. A power screw 88 is free to rotate in the housing 86. The opposite ends of the power screw are formed with external threads 94, 96 of opposite hand. The power screw is engaged with the coupler 74 by threaded engagement of the thread 96 with an internal thread 98 in distal end of the coupler.

The distraction component 84 includes bone attachment plates 100, 102 which have internally threaded portions (not visible in the drawings) which extend through the slot 87 and make threaded engagement with the respective threads 94, 96. It will be understood that if the power screw is rotated in one direction about its axis, the bone distraction plates will be moved towards one another, and if the power screw is rotated in the opposite direction the bone distraction plates will be moved apart from one another, i.e. distracted. The arrangement is such that the threaded engagement between the power screw 88 and the coupler 74 will tighten further if the power screw is rotated in the direction to cause distraction of the bone attachment plates.

The numeral 104 indicates a hexagonal section engagement formation, at the opposite, proximal end of the drive assembly 12, which can be engaged by a suitable wrench or other tool for the purposes of applying rotary torque to the drive assembly. The formation 104 in this embodiment has a male end, similar to the male end 20 of a drive element 14, which is connected to the endmost drive element 14.2 at the proximal end of the assembly 12. This connection is achieved in the same way that connections are made between adjacent drive elements, i.e. a snap fit connection.

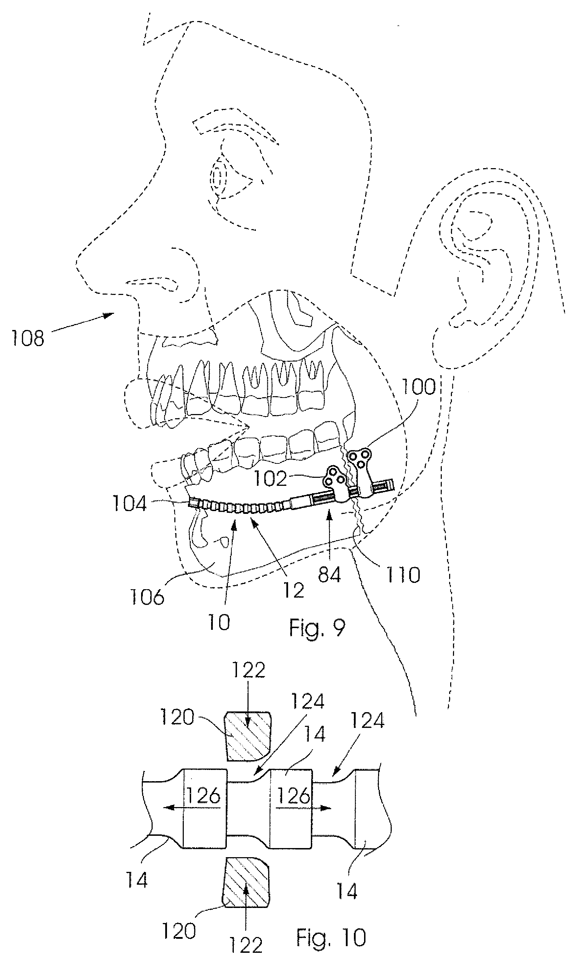

Figure 9 illustrates the use of the distraction apparatus 10 to lengthen the mandible 106 of a patient 108 suffering from microagnathia. A corticotomy has been performed to break the mandibular bone at 110. The distraction component is placed in an incision in the soft tissue in the lower gum and the bone attachment plates 100, 102 are connected to the bone on opposite sides of the break. This is achieved by means of screws 112, 114 passing through holes 116, 118 in the plates and into the bone.

The flexible drive assembly 12 is flexed as necessary to extend forwardly and around the mandible from the distraction component 84. In practice, the drive assembly 12 is sutured into an incision made for the purpose in the soft tissue of the lower gum, with only its proximal end, including the formation 104, projecting forwardly out of the incision.

The overall flexibility of the drive assembly 12 allows the assembly to be flexed to suit a wide variety of different shapes. In the illustrated case, this enables the assembly to be used in a variety of different mouths in which the jawbones may be of different size or shape.

Once the apparatus has been installed in the manner described above, and a prescribed period of latency has passed, actual distraction can commence. At regular time intervals a wrench or other tool is engaged with the formation 104 and is operated to rotate the formation and hence the drive assembly 12. The drive assembly in turn transmits the same rotation to the connection element 60. The connection element transmits the same rotation to the coupler 74 through frictional engagement between the threads 66 and 76 and between the shoulder 72 and the proximal end surface of the coupler. The coupler in turn transmits the same rotation to the power screw 88.

The direction of the rotary torque applied to the formation 104 and transmitted to the power screw 88 is in the sense required to distract the bone attachment plates 100, 102. The bone portions on either side of the break 110 are accordingly distracted, i.e. moved apart from one another.

In a typical distraction procedure, a certain amount of rotation, corresponding to a certain amount of distraction, is applied to the formation at regular time intervals. For example, one full rotation, corresponding to 1 mm of distraction, may be applied to the formation once per day. The procedure may for example be repeated every day for, say nine days, corresponding to a total of 9mm distraction.

After the required amount of distraction has taken place, the flexible drive assembly is disconnected from the distraction component 84. This is achieved by applying reverse torque to the formation 104. The reverse torque which is applied must be sufficient to cause the rib 68 to unseat, i.e. unsnap, from the groove 78, whereafter further reverse torque serves to unscrew the connection element 60 from the coupler 74. After disconnection of the flexible drive assembly from the distraction component, the drive assembly may be withdrawn and the gum in which it was laid is allowed to heal. The soft tissue of the gum may if necessary be sutured closed over the distraction component, which is, with the bone attachment plates still in the distracted condition, left in place for a period of time sufficient for bone growth, i.e. ossification, to take place in the distracted break 110. After the required healing period, for example six to ten weeks, the distraction component is disconnected from the bone and removed through an incision in the gum.

The fact that a substantial, predetermined reverse torque has to be applied to the formation 104 in order to disconnect the flexible drive assembly from the distraction component is considered advantageous because this will make it more difficult for the patient himself inadvertently to achieve a disconnection. It is envisaged that in practice the patient or his assistant will be supplied with a unidirectional wrench or other tool which can be used to achieve distraction but which cannot be used to apply reverse torque. Only the clinician will have access to a tool able to apply torque in both directions.

When compared to the known KLS Martin system described above, the distraction apparatus described above has a number of advantages, as follows:
1. The spring-type flexible drive of the KLS Martin system cannot be detached from the actual distraction components. As described above, this allows infection to migrate along the springs of the flexible drive during the healing period. In contrast the flexible drive assembly 12 is detachable from the distraction component 84 after distraction, as described above, thereby allowing the soft tissue to be sutured closed and preventing ingress of infection. In other known systems, it is possible to detach the drive assembly from the distraction components but this generally requires a surgical intervention to expose the drive assembly for detachment thereof. In the present invention, once sufficient reverse torque has been applied to unsnap the connection element from the coupler, the drive assembly can simply be drawn lengthways out of the soft tissue in which it is embedded.
2. In the KLS Martin system, rotation can be lost during distraction as a result of spring wind-up. As the flexible drive assembly of the present invention is composed of rigid, interconnected drive elements, there is no such loss of rotation, and the user can be sure that a given amount of rotation applied to the formation 104 is fully transferred to the power screw, thereby achieving a predetermined distraction.
3. The springs of the KLS Martin system do not allow for any reverse torque to be applied. In the present invention, torque applied at a lower level than that required to unsnap the connection element from the coupler can be used to rotate the power screw in a sense to move the bone attachment plates closer to one another. This may for example be appropriate in a situation where excessive distraction has inadvertantly been applied.
4. The drive of the KLS Martin system will always have a tendency to assume a straight configuration because of the inherent resilience of the springs. Where the drive has been laid in a curve in an incision in soft tissue, the drive will accordingly have a tendency to break through the suturing which closes the incision. In the present invention, there is no inherent resilience trying to straighten the drive assembly which will remain passively at any selected curvature through the action of the wire 54.
5. The drive assembly of the present invention can be made to have any practical length, merely by adding or removing drive elements. In the KLS system the drive can only be shortened by cutting the springs. This means that the drive of the KLS system must be custom made for each application.
6. It is believed that the drive assembly of the present invention can be made more economically than the drive of the KLS Martin system, *inter alia* because there is no requirement for welding.

In the illustrated example there is, as explained above, the facility to disconnect the entire drive assembly 12 from the distraction component 14 after a required amount of distraction has taken place. Figure 10 shows how individual drive elements 14 the drive assembly can be disconnected from one another at any selected location along the length of the assembly.

In Figure 10 the numerals 120 indicate the opposing jaws of a tool which can be moved forcibly towards one another by operation of the tool. The tool may for example be a pliers or pincers. The opposing jaws are shaped to engage in the gap 124 formed by the waist 48 of a drive element between the enlarged end sections 38 of any two selected, connected drive elements. The jaw shape is selected such that as the jaws are forced together, as indicated by the numerals 122, they apply oppositely directed components of force, indicated by the numerals 126, to the connected drive elements. If these components of force are sufficiently large, the rib 26 of one drive element can be unsnapped from the groove 44 of the other drive element, thereby disconnecting the drive assembly 12 at the selected location. If an internal, shape-retaining wire 54 has been used, the wire can, after separation of the drive elements, be cut using another suitable tool, for example side cutters.

To facilitate the disconnection of the drive assembly using this technique, the surfaces 128, 130 (Figure 4) of the respective drive elements may be shaped such that a sufficiently large separating force is generated when jaws 120 of appropriate shape are forced together in the gap 124. It will however be understood that, in practice, use can be made of any tool capable of acting in the gap 124 to exert a sufficiently large separating force to the end sections 38 of the connected elements 14.

## Claims

1. A bone distraction apparatus (10) including a flexible drive assembly (12), **characterised in that** the flexible drive assembly (12) comprises a plurality of identical drive elements (14) connected to one another in series, each drive element having a body (16) with a central axis (18) and male (20) and female (22) ends, the male end including a laterally projecting rib (26) of non-circular cross-section and the female end defining a socket (36) with a laterally extending groove (44) therein which is of non-circular cross-section, wherein the drive elements are connected to one another in series by location of the male end of one element in the series in the socket at the female end of a neighbouring element in the series with the rib of the male end received as a snap fit in the groove of the socket, the male ends and sockets being configured to allow limited pivotal movement to take place between adjacent, connected elements, in each case about an axis transverse to the central axes of the elements, while also allowing the elements to transmit rotational torque in either direction from one to the other.

2. A bone distraction apparatus (10) according to claim 1 wherein the male end (20) of each drive element (14) of the flexible drive assembly (12) has oppositely inclined flanks (28, 30) on either side of the projecting rib (26), the flanks defining a non-circular cross-section.

3. A bone distraction apparatus (10) according to claim 2 wherein the rib (26) and flanks (28, 30) of the male end (20) of each drive element (14) of the flexible drive assembly (12) have a polygonal, preferably hexagonal, cross-section.

4. A bone distraction apparatus (10) according to claim 3 wherein the socket (36) and groove (44) of the female end (22) of each drive element (14) of the flexible drive assembly (12) have a polygonal, preferably hexagonal, cross-section.

5. A bone distraction apparatus (10) according to claim 4 wherein transverse dimensions of the socket (36) and groove (44) are greater than corresponding transverse dimensions of the rib (26) and flanks (28, 30) by an amount which allows pivotal movement to take place between connected elements (14) of the flexible drive assembly (12) while still ensuring rotational interference between the elements for torque transmission purposes.

6. A bone distraction apparatus (10) according to claim 5 wherein each element (14) of the flexible drive assembly (12) has an axial passage (24) through it, the axial passages of the individual elements combining to form a continuous passage through the connected elements of the drive assembly.

7. A bone distraction apparatus (10) according to claim 6 wherein the drive assembly (12) includes an elongate, bendable member, preferably of wire (54), which extends through the continuous passage to maintain a desired angulation of the assembly.

8. A bone distraction apparatus (10) according to any one of the preceding claims wherein the elements (14) are configured such that each element in the series can be axially misaligned by at least 5°, preferably about 6.5°, relative to adjacent elements.

9. A bone distraction apparatus (10) according to any one of the preceding claims wherein the apparatus includes a distraction component (84) including a rotatable power screw (88) which has respective ends carrying threads (94, 96) of opposite hand and bone attachment members (100, 102) engaged in threaded manner with the respective ends of the power screw such that rotation of the power screw moves the bone attachment members towards or away from one another, and wherein a first, distal end of the flexible drive assembly (12) is connected in a rotation-transmitting manner to the power screw.

10. A bone distraction apparatus (10) according to claim 9 wherein the opposite, proximal end of the flexible drive assembly (12) carries a formation (104) engagable by a rotary drive tool, whereby the drive tool can be operated to rotate the drive assembly, and hence the power screw (88), via the drive assembly.

11. A bone distraction apparatus (10) according to either one of claims 9 or 10 wherein the apparatus includes a coupling means (66, 74) which connects the flexible drive assembly (12) to the power screw (88) in a manner which allows rotational torque to be transmitted to the power screw in a first direction corresponding to distraction of the bone attachment members (100, 102), but which disconnects the drive assembly from the power screw if rotational torque of predetermined magnitude is applied to the drive assembly in an opposite, second direction.

12. A bone distraction apparatus according to claim 11 wherein the coupling means includes a coupler (74) connectable to an end of the power screw (88) and a connection element (66) at the distal end of the drive assembly (12) to which an endmost one (14.1) of the drive elements (14) is connectable and which is itself connectable to the coupler in a manner to transmit torque in the first direction and which disconnects from the coupler when torque of the predetermined magnitude is applied to the drive assembly in the second direction.

13. A bone distraction apparatus (10) according to claim 12 wherein the coupler (74) includes a threaded socket at a distal end thereof into which one end of the power screw (88) is threaded in order to connect the coupler to the power screw at a threaded connection.

14. A bone distraction apparatus (10) according to claim 13 wherein the threaded connection is arranged to tighten when torque, corresponding to distraction of the bone attachment members (100, 102), is applied.

15. A bone distraction apparatus (10) according to any one of claims 12 to 14 wherein the coupler (74) and connection element (66) make threaded engagement with one another, one of them including a radial rib (68) which snaps into a radial groove (78) in the other of them when they are threaded together, and the configuration being such that the rib unsnaps from the groove when the predetermined magnitude of torque is applied in the second direction.

## Patentansprüche

1. Knochendistraktionsvorrichtung (10), die eine flexible Antriebsanordnung (12) aufweist, **dadurch gekennzeichnet, dass** die flexible Antriebsanordnung (12) mehrere identische Antriebselemente (14) umfasst, die in einer Reihe miteinander verbunden sind, wobei jedes Antriebselement einen Körper (16) mit einer Mittelachse (18) und einem Einschubende (20) und einem Aufnahmeende (22) aufweist, wobei das Einschubende eine seitlich hervorstehende Rippe (28) mit einem nicht-kreisförmigen Querschnitt aufweist und das Aufnahmeende eine Steckaufnahme (36) mit einer seitlich verlaufenden Nut (44) darin definiert, die von nicht-kreisförmigem Querschnitt ist, wobei die Antriebselemente in einer Reihe miteinander verbunden sind, indem das Einschubende eines Elements in der Reihe in die Steckaufnahme am Aufnahmeende eines benachbarten Elements in der Reihe angeordnet wird, wobei die Rippe des Einschubendes einrastend in der Nut der Steckaufnahme aufgenommen wird, wobei die Einschubenden und die Steckaufnahmen so ausgebildet sind, dass eine begrenzte Schwenkbewegung zwischen benachbarten, verbundenen Elementen jeweils um eine Achse, die quer zu den Mittelachsen der Elemente verläuft, stattfinden kann, während außerdem gestattet wird, dass die Elemente ein Drehmoment in jeder Richtung von einem zum anderen übertragen.

2. Knochendistraktionsvorrichtung (10) nach Anspruch 1, wobei das Einschubende (20) eines jeden Antriebselements (14) der flexiblen Antriebsanordnung (12) entgegengesetzt geneigte Flanken (28, 30) auf jeder Seite der hervorstehenden Rippe (26) hat, wobei die Flanken einen nicht-kreisförmigen Querschnitt definieren.

3. Knochendistraktionsvorrichtung (10) nach Anspruch 2, wobei die Rippe (26) und die Flanken (28, 30) des Einschubendes (20) eines jeden Antriebselements (14) der flexiblen Antriebsanordnung (12) einen polygonalen, bevorzugt hexagonalen, Querschnitt aufweisen.

4. Knochendistraktionsvorrichtung (10) nach Anspruch 3, wobei die Steckaufnahme (30) und die Nut (44) des Aufnahmeendes (22) eines jeden Antriebselements (14) der flexiblen Antriebsanordnung (12) einen polygonalen, bevorzugt hexagonalen, Querschnitt aufweisen.

5. Knochendistraktionsvorrichtung (10) nach Anspruch 4, wobei die Querabmessungen der Steckaufnahme (36) und der Nut (44) um einen Betrag größer sind als entsprechende Querabmessungen der Rippe (28) und der Flanken (28, 30), der eine Schwenkbewegung zwischen verbundenen Elementen (14) der flexiblen Antriebsanordnung (12) gestattet, während weiterhin eine Rotationsmitnahme zwischen den Elementen zum Zweck der Drehmomentübertragung möglich ist.

6. Knochendistraktionsvorrichtung (10) nach Anspruch 5, wobei durch jedes Element (14) der flexiblen Antriebsanordnung (12) eine axiale Passage (24) führt, wobei sich die axialen Passagen der einzelnen Elemente zu einer durchgängigen Passage durch die verbundenen Elemente der Antriebsanordnung kombinieren.

7. Knochendistraktionsvorrichtung (10) nach Anspruch 6, wobei die Antriebsanordnung (12) ein längliches, biegsames Funktionsglied, bevorzugt aus Draht (54), aufweist, das sich durch die durchgängige Passage erstreckt, um eine gewünschte Winkelung der Anordnung beizubehalten.

8. Knochendistraktionsvorrichtung (10) nach einem der vorangehenden Ansprüche, wobei die Elemente (14) so ausgebildet sind, dass jedes Element in der Reihe axial um mindestens 5°, bevorzugt etwa 6,5°, relativ zu benachbarten Elementen fehlausgerichtet werden kann.

9. Knochendistraktionsvorrichtung (10) nach einem der vorangehenden Ansprüche, wobei die Vorrichtung eine Distraktionskomponente (84) aufweist, die eine drehbare Kraftspindel (88) aufweist, die jeweilige Enden aufweist, an denen sich Gewindegänge (94, 96) von gegenläufigem Drehsinn befinden, und Knochenbefestigungsglieder (100, 102) aufweist, die mit den jeweiligen Enden der Kraftspindel dergestalt in Schraubeingriff stehen, dass eine Rotation der Kraftspindel die Knochenbefestigungsglieder aufeinander zu oder voneinander fort bewegt, und wobei ein erstes, distales Ende der flexiblen Antriebsanordnung (12) in einer rotationsübertragenden Weise mit der Kraftspindel verbunden ist.

10. Knochendistraktionsvorrichtung (10) nach Anspruch 9, wobei das gegenüberliegende, proximale Ende der flexiblen Antriebsanordnung (12) eine Formation (104) aufweist, die von einem Drehantriebswerkzeug in Eingriff genommen werden, wobei das Antriebswerkzeug dafür verwendet werden kann, die Antriebsanordnung und folglich die Kraftspindel (88) mittels der Antriebsanordnung zu drehen.

11. Knochendistraktionsvorrichtung (10) nach einem der Ansprüche 9 oder 10, wobei die Vorrichtung ein Kopplungsmittel (66, 74) aufweist, das die flexible Antriebsanordnung (12) mit der Kraftspindel (88) in einer Weise verbindet, die eine Übertragung von Drehmoment zu der Kraftspindel in einer ersten Richtung erlaubt, die der Distraktion der Knochenbefestigungsglieder (100, 102) entspricht, die aber die Antriebsanordnung von der Kraftspindel trennt, wenn ein Drehmoment von einer vorgegebenen Größenordnung an die Antriebsanordnung in einer entgegengesetzten, zweiten Richtung angelegt wird.

12. Knochendistraktionsvorrichtung (10) nach Anspruch 11, wobei das Kopplungsmittel einen Koppler (74) aufweist, der mit einem Ende der Kraftspindel (88) verbunden werden kann, und ein Verbindungselement (66) am distalen Ende der Antriebsanordnung (12) aufweist, mit dem ein am hintersten Ende befindliches (14.1) der Antriebselemente (14) verbunden werden kann und das seinerseits mit dem Koppler in einer Weise verbunden werden kann, um ein Drehmoment in der ersten Richtung zu übertragen, und das sich von dem Koppler trennt, wenn ein Drehmoment der vorgegebenen Größenordnung an die Antriebsanordnung in der zweiten Richtung angelegt wird.

13. Knochendistraktionsvorrichtung (10) nach Anspruch 12, wobei der Koppler (74) eine mit einem Gewinde versehene Steckaufnahme an seinem distalen Ende aufweist, in die ein Ende der Kraftspindel (88) eingeschraubt wird, um den Koppler an einer Gewindeverbindung mit der Kraftspindel zu verbinden.

14. Knochendistraktionsvorrichtung (10) nach Anspruch 13, wobei die Gewindeverbindung dafür ausgelegt ist, sich festzuziehen, wenn ein Drehmoment angelegt wird, das einer Distraktion der Knochenbefestigungsglieder (100, 102) entspricht.

15. Knochendistraktionsvorrichtung (10) nach einem der Ansprüche 12 bis 14, wobei der Koppler (74) und das Verbindungselement (66) miteinander in Schraubeingriff gelangen, wobei eines von ihnen eine radiale Rippe (68) aufweist, die in eine radiale Nut (78) des anderen von ihnen einrastet, wenn sie miteinander verschraubt werden, und wobei die Ausgestaltung dergestalt ist, dass die Rippe aus der Nut ausrastet, wenn die vorgegebene Größenordnung an Drehmoment in der zweiten Richtung angelegt wird.

## Revendications

1. Appareil de distraction osseuse (10) incluant un ensemble d'entraînement flexible (12), **caractérisé en ce que** l'ensemble d'entraînement flexible (12) comprend une pluralité d'éléments d'entraînement identiques (14) raccordés les uns aux autres en série, chaque élément d'entraînement ayant un corps (16) avec un axe central (18) et des extrémités mâle (20) et femelle (22), l'extrémité mâle incluant une nervure faisant saillie latéralement (26) de section non circulaire et l'extrémité femelle définissant une douille (36) avec une rainure s'étendant latéralement (44) à l'intérieur qui est de section non circulaire, les éléments d'entraînement étant raccordés les uns aux autres en série par localisation de l'extrémité mâle d'un élément dans la série dans la douille à l'extrémité femelle d'un élément voisin dans la série avec la nervure de l'extrémité mâle reçue par emboîtement dans la rainure de la douille, les extrémités mâles et les douilles étant configurées pour permettre un mouvement pivotant limité entre des éléments raccordés adjacents, dans chaque cas autour d'un axe transversal aux axes centraux des éléments, tout en permettant également aux éléments de transmettre un couple de rotation dans l'une ou l'autre direction l'un par rapport à l'autre.

2. Appareil de distraction osseuse (10) selon la revendication 1, dans lequel l'extrémité mâle (20) de chaque élément d'entraînement (14) de l'ensemble d'entraînement flexible (12) a des flancs inclinés à l'opposé (28, 30) de part et d'autre de la nervure en saillie (26), les flancs définissant une section non circulaire.

3. Appareil de distraction osseuse (10) selon la revendication 2, dans lequel la nervure (26) et les flancs (28, 30) de l'extrémité mâle (20) de chaque élément d'entraînement (14) de l'ensemble d'entraînement flexible (12) ont une section polygonale, de préférence hexagonale.

4. Appareil de distraction osseuse (10) selon la revendication 3, dans lequel la douille (36) et la rainure (44) de l'extrémité femelle (22) de chaque élément d'entraînement (14) de l'ensemble d'entraînement flexible (12) ont une section polygonale, de préférence hexagonale.

5. Appareil de distraction osseuse (10) selon la revendication 4, dans lequel des dimensions transversales de la douille (36) et de la rainure (44) sont plus grandes que des dimensions transversales correspondantes de la nervure (26) et des flancs (28, 30) d'une quantité qui permet à un mouvement pivotant de se dérouler entre des éléments raccordés (14) de l'ensemble d'entraînement flexible (12) tout en assurant encore une interférence de rotation entre les éléments à des fins de transmission de couple.

6. Appareil de distraction osseuse (10) selon la revendication 5, dans lequel chaque élément (14) de l'ensemble d'entraînement flexible (12) est traversé par un passage axial (24), les passages axiaux des éléments individuels se combinant pour former un passage continu à travers les éléments raccordés de l'ensemble d'entraînement.

7. Appareil de distraction osseuse (10) selon la revendication 6, dans lequel l'ensemble d'entraînement (12) inclut un organe allongé et pouvant être fléchi, de préférence en fil (54), qui s'étend à travers le passage continu pour maintenir une angulation souhaitée de l'ensemble.

8. Appareil de distraction osseuse (10) selon l'une quelconque des revendications précédentes, dans lequel les éléments (14) sont configurés pour que chaque élément dans la série puisse être désaligné axialement d'au moins 5°, de préférence d'environ 6,5°, par rapport à des éléments adjacents.

9. Appareil de distraction osseuse (10) selon l'une quelconque des revendications précédentes, dans lequel l'appareil inclut un composant de distraction (84) incluant une vis de puissance rotative (88) qui comporte des extrémités respectives portant des filets (94, 96) de côté opposé et des organes de fixation osseuse (100, 102) mis en prise de manière filetée avec les extrémités respectives de la vis de puissance de telle sorte qu'une rotation de la vis de puissance rapproche ou éloigne les organes de fixation osseuse les uns des autres, et une première extrémité distale de l'ensemble entraînement flexible (12) étant raccordée à la vis de puissance de manière à transmettre une rotation.

10. Appareil de distraction osseuse (10) selon la revendication 9, dans lequel l'extrémité proximale opposée de l'ensemble entraînement flexible (12) porte une formation (104) pouvant être mise en prise par un outil d'entraînement rotatif de sorte que l'outil d'entraînement puisse être actionné pour faire tourner l'ensemble d'entraînement, et donc la vis de puissance (88), via l'ensemble d'entraînement.

11. Appareil de distraction osseuse (10) selon l'une ou l'autre des revendications 9 ou 10, dans lequel l'appareil inclut un moyen de couplage (66, 74) qui raccorde l'ensemble d'entraînement flexible (12) à la vis de puissance (88) d'une manière qui permet de transmettre un couple de rotation à la vis de puissance dans un premier sens correspondant à la distraction des organes de fixation osseuse (100, 102), mais qui déconnecte l'ensemble d'entraînement de la vis de puissance si un couple de rotation de grandeur prédéterminée est appliqué à l'ensemble d'entraînement dans un second sens opposé.

12. Appareil de distraction osseuse selon la revendication 11, dans lequel le moyen de couplage inclut un coupleur (74) pouvant être raccordé à une extrémité de la vis de puissance (88) et un élément de raccord (66) à l'extrémité distale de l'ensemble d'entraînement (12) auquel un élément le plus extrême (14.1) des éléments d'entraînement (14) peut être raccordé et qui lui-même peut être raccordé au coupleur de manière à transmettre un couple dans le premier sens et qui se débranche du coupleur lorsqu'un couple de la grandeur prédéterminée est appliqué à l'ensemble d'entraînement dans le second sens.

13. Appareil de distraction osseuse (10) selon la revendication 12, dans lequel le coupleur (74) inclut une douille filetée à l'une de ses extrémités distales dans laquelle une extrémité de la vis de puissance (88) est filetée afin de raccorder le coupleur à la vis de puissance au niveau d'un raccord fileté.

14. Appareil de distraction osseuse (10) selon la revendication 13, dans lequel le raccord fileté est agencé pour être serré lorsqu'un couple, correspondant à la distraction des organes de fixation osseuse (100, 102), est appliqué.

15. Appareil de distraction osseuse (10) selon l'une quelconque des revendications 12 à 14, dans lequel le coupleur (74) et l'élément de raccord (66) réalisent une mise en prise filetée l'un avec l'autre, l'un d'entre eux incluant une nervure radiale (68) qui s'emboîte dans une rainure radiale (78) dans l'autre d'entre eux lorsqu'ils sont filetés ensemble, et la configuration étant telle que la nervure se détache de la rainure lorsque la grandeur de couple prédéterminée est appliquée dans le second sens.
